Europäisches Patentamt

European Patent Office

Office européen des brevets

(19)

(11) **EP 1 321 771 A1**

(12) **EUROPÄISCHE PATENTANMELDUNG**

(43) Veröffentlichungstag:
**25.06.2003 Patentblatt 2003/26**

(51) Int Cl.$^7$: **G01N 33/68**, G01N 33/58, G01N 33/557

(21) Anmeldenummer: **01130633.9**

(22) Anmeldetag: **21.12.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(71) Anmelder: **Evotec Analytical Systems GmbH**
**40699 Erkrath (DE)**

(72) Erfinder:
• **Hecks, Birgit, Dr.**
**40237 Düsseldorf (DE)**

• **Pitschke, Martin, Dr.**
**42555 Velbert-Langenberg (DE)**
• **Storm, Claudia**
**50999 Köln (DE)**
• **Langer, Jessica**
**40789 Monheim (DE)**

(74) Vertreter:
**Meyers, Hans-Wilhelm, Dr.Dipl.-Chem. et al**
**Patentanwälte**
**von Kreisler-Selting-Werner**
**Postfach 10 22 41**
**50462 Köln (DE)**

(54) **Verfahren zur qualitativen und/oder quantitativen Bestimmung von Aggregaten**

(57) Verfahren zur qualitativen und/oder quantitativen Bestimmung von Aggregaten, dadurch gekennzeichnet, dass die Assoziation mindestens eines Proteins, Proteinfragments, Proteinanalogons, insbesondere einer Proteinen und/oder Proteinfragmenten entsprechenden Struktur, Peptids, Peptidfragments und/oder Peptidanalogons, insbesondere einer Peptiden und/oder Peptidfragmenten entsprechenden Struktur, als Sonde an mindestens ein Aggregat bestimmt wird, bevor eine Selbstaggregation der mindestens einen Sonde überwiegt.

**Abbildung 1**

EP 1 321 771 A1

**Beschreibung**

Die vorliegende Erfindung betrifft ein Verfahren zur qualitativen und/oder quantitativen Bestimmung von Aggregaten, die insbesondere aus zur Aggregation neigenden Molekülen, beispielsweise Proteinen, Proteinfragmenten, Proteinanaloga, insbesondere Proteinen und/oder Proteinfragmenten entsprechenden Strukturen, Peptiden, Peptidfragmenten und/oder Peptidanaloga, insbesondere Peptiden und/oder Peptidfragmenten entsprechenden Strukturen bestehen.

Generell ist die Peptidproduktion, insbesondere die erfolgreiche Synthese von zur Aggregation neigenden Peptiden, oft mit großen Schwierigkeiten verbunden *(Nilsson M.R. et al., (2001) Synthesis and purification of amyloidogenic peptides*, *Anal Biochem 288 (1): 76-82)*, da sie, wie beispielsweise Peptidhormone (Insulin, Glucagon, ANF), konzentrations- und umgebungsabhängig in Aggregate konvertiert werden.

Vergleichbare Probleme treten auch bei der gentechnischen Herstellung von Proteinen, beispielsweise bei der Exprimierung in Prokaryoten oder Eukaryoten, auf. Daher ist es oft schwierig, lösliche und aktive Proteine zu erhalten. Vielfach führt zudem Überexprimierung zur Proteinablagerung, wie z. B. in Prokaryoten zur Produktion von "inclusion bodies". Es handelt sich dabei um unlösliche Aggregate von nicht korrekt gefaltetem Protein *(Parkar, A.A., (2000) Large-scale expression, refolding, and purification of the catalytic domain of human macrophage metalloelastase (MMP-12) in Escherichia coli, Protein Expr. Purif. 20(2): 152-161)*, die im Laufe der Proteinaufreinigung solubilisiert oder entfernt werden müssen.

Die Aggregatbildung stellt auch ein Problem für die Herstellung von Medikamenten/ Therapeutika dar, da insbesondere Peptide oder Proteine zunehmend an Bedeutung als Therapeutika gewinnen. Beispielsweise sind hier Peptidhormone als Therapeutika und Antikörper als Therapeutika und Diagnostika, z. B. für non-Hodgkin Lymphoma oder Crohn's disease, zu nennen *(Borrebaeck CA et al., (2001) Human therapeutic antibodies, Curr Opin Pharmacol 1(4): 404-8)*. Zudem werden Peptide oder Proteine in diesen Therapeutika häufig in sehr hohen Konzentrationen verwendet, was die Wahrscheinlichkeit der Bildung von Aggregaten noch zusätzlich erhöht. Darüber hinaus wird vermutet, dass Aggregate beispielsweise bei der Injektion von o. g. Therapeutika, insbesondere von Antikörpern, an der Injektionsstelle beispielsweise Schwellungen hervorrufen und somit einen negativen medizinischen Effekt aufweisen. Eine Kontrolle des Aggregatgehaltes ist daher zur Überwachung der Qualität eines Medikamentes/ Therapeutikums unbedingt notwendig.

Der Aggregatgehalt von Proteinen und/oder Peptiden verursacht auch Probleme in der Forschung. So zeigte sich beispielsweise, dass Verunreinigungen mit Peptidaggregaten für die unterschiedlichen Ergebnisse von Versuchen mit neuronalen Zellkulturen bei Inkubationen mit Amyloid-β-Peptiden verantwortlich waren. Je nach verwendeter Charge und je nach Hersteller wurden unterschiedliche Ergebnisse erhalten *(May P. C*., *et al. (1992) beta-Amyloid peptide in vitro toxicity: lotto-lot variability. Neurobiol Aging 3: 605-607)*. Dabei hatten die Peptidaggregate einen neurotoxischen und somit negativen Einfluss auf die Überlebensrate der Zellen.

Es ist daher auch bei diesen isolierten Proteinen oder Petiden, insbesondere synthetisch hergestellten Peptiden und gentechnisch synthetisierten Proteinen, wichtig, ihren tatsächlichen Gehalt an Aggregaten zu bestimmen.

Gegenwärtig erfolgt die Beurteilung der Qualität von synthetisierten Peptiden, d.h. die Bestimmung des Anteils an Aggregaten, beispielsweise durch HPLC und Massenspektrometrieverfahren (MALDI). Zum Nachweis amyloider Fibrillen werden FTIR (Fourier transform infrared spectroscopy), CD (circular dicroism) und EM (Elektronenmikroskopie) angewendet *(Bouchard M., et al (2000), Formation of Insulin amyloid fibrils followed by FTIR simultaneously with CD and electron microscopy. Protein Sci 9(10): 1960-1967)*. Die hier aufgeführten Verfahren zur Bestimmung des Anteils an Aggregaten erlauben zwar eine quantitative Bestimmung eines Gehaltes von bis zu 1 ng Aggregat, allerdings sind sie häufig sehr zeit- und kostenintensiv und bedürfen oftmals einer aufwendigen Probenvorbereitung, die den Aggregatanteil der Probe beeinflussen kann. Sie sind daher, wenn überhaupt, nur im Labormassstab befriedigend anwendbar.

Bei der Aufreinigung von Proteinen aus "inclusion bodies" erfolgt der Nachweis von Proteinaggregation häufig über eine Streuungsmessung (dynamische Lichtstreuung) der Lösung und vergleichend dazu die Bestimmung der Absorption bei 400 nm (U.S. *Dept Commerce/NOAA/NMFS/NWFSC/Molecular Biology Protocols*: *Preparation of active proteine from inclusion bodies, Laurant Vuillard & Alasdair Freeman, University of St Andrews U.K.)*.

Generell sind Methoden, wie Streuungsmessungen und Absorptionsbestimmungen, zwar vergleichsweise schnell durchzuführen, allerdings sind sie relativ unspezifisch und unempfindlich, d.h. der Aggregatanteil muss mindestens 1% des Proteinanteils betragen, um nachweisbar zu sein. Zudem beeinflusst bei diesen Verfahren die Eigentrübung des Mediums oder das Vorhandensein anderer Nichtaggregatkomponenten in der Probe das Messergebniss und führt zu ungenauen oder sogar falschen Ergebnissen. Somit ist dieses Verfahren nur bedingt für einen qualitativen Aggregatnachweis, allerdings nicht für einen quantitativen Nachweis, z. B. in der Kontrolle von Therapeutika (Medikamente), einsetzbar.

Gleiches gilt für gelelektrophoretische Nachweisverfahren, die ebenfalls nur eine geringe Sensitivität aufweisen und somit nur für den qualitativen Nachweis von höheren Aggregat-Konzentrationen geeignet sind. Zudem müssen die Aggregate vor der gelelektrophoretischen Analyse teilsolubilisiert bzw. denaturiert werden. Der Aggregatnachweis erfolgt somit nur indirekt über die unspezifische Anfärbung der einzelnen Aggregatkomponenten. Zudem stört diese aufwendige Probenvorbereitung, insbesondere Umpufferung, Änderung der Salzbedingungen, pH oder Aufkonzentration, das in der zu testenden Probe bestehende Gleichgewicht und kann den Aggregationsgrad erheblich beeinflussen. Die maximale Sensitivität, die mit gelelektrophoretischen Methoden erreicht werden kann, liegt daher abhängig von der Färbetechnik nur bei maximal 1 ng, beispielsweise für die Silberfärbung.

Alle hier aufgeführten Verfahren sind aufgrund des hohen Aufwandes entweder nur im Labormassstab befriedigend anwendbar oder zu unspezifisch und daher nur bedingt für eine qualitative Bestimmung des Aggregatgehaltes, aufgrund fehlender Sensitivität und insbesondere Spezifität nicht für eine quantitiative Bestimmung geeignet. Sie sind daher auch beispielsweise zur Überwachung einer industriellen Synthese von zur Aggregation neigenden Molekülen, z. B. Proteinen, Proteinfragmenten, Proteinanaloga, insbesondere Protein und/oder Proteinfagmenten entsprechenden Strukturen und/oder Peptiden, Peptidfragmenten und/oder Peptidanaloga, insbesondere Peptiden und/oder Peptidfragmenten entsprechenden Strukturen oder auch zur Qualitätskontrolle von Therapeutika, nicht geeignet.

Aufgabe der vorliegenden Erfindung ist es daher, ein hoch spezifisches und sensitives Verfahren zur qualitativen und quantitativen Bestimmung von Aggregaten zu schaffen, das die o. g. Nachteile nicht aufweist. Dieses Verfahren sollte zudem insbesondere für den routinemäßigen Einsatz in der Qualitätskontrolle geeignet sein. Darüber hinaus ist es ebenfalls eine Aufgabe der vorliegenden Erfindung, ein Verfahren zur Identifizierung und Optimierung von Substanzen, die eine Aggregatbildung beeinflussen können, bereitzustellen.

Gelöst wurden diese Aufgaben durch ein Verfahren zur qualitativen und quantitativen Bestimmung von Aggregaten mit den Merkmalen des Anspruch 1 und ein Verfahren zur Identifizierung und Optimierung von Substanzen, die eine Aggregatbildung beeinflussen können, mit den Merkmalen des Anspruch 15. Bevorzugte Ausführungsformen sind in den Unteransprüchen beschrieben.

| Abbildung 1 | zeigt das Prinzip der Keim-induzierten Multimerisierung. |
| Abbildung 2 | zeigt das FIDA-Signal in Abhängigkeit von der Konzentration an Protein-Aggregaten. |
| Abbildung 3 | zeigt die Ergebnisse eines Experiments zur Untersuchung unterschiedlicher Chargen eines Peptids. |
| Abbildung 4 | zeigt die Ergebnisse einer Untersuchung unterschiedlicher Chargen eines Antikörpers. |

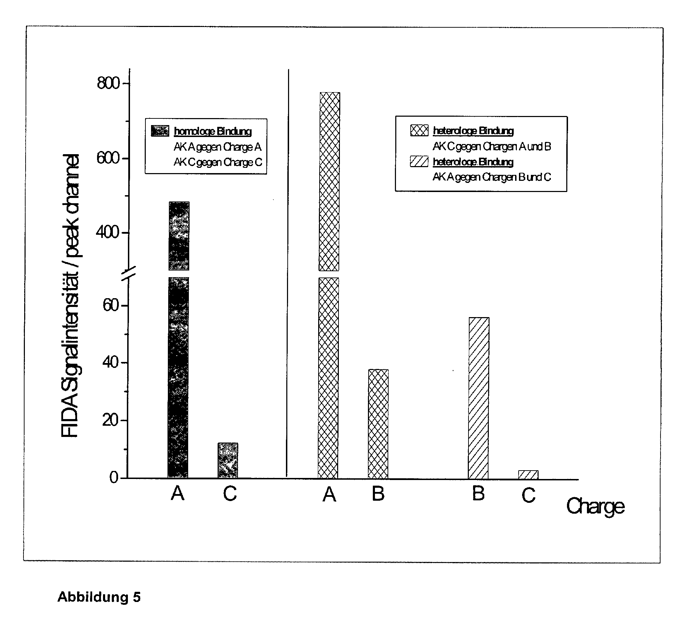

Abbildung 5    zeigt die Ergebnisse von homologer und heterologer Antikörperdetektion.

Das erfindungsgemäße Verfahren zur qualitativen und/oder quantitativen Bestimmung von Aggregaten, zeichnet sich insbesondere dadurch aus, dass die Assoziation mindestens eines Proteins, Proteinfragments, Proteinanalogons, insbesondere einer Proteinen und/oder Proteinfragmenten entsprechenden Struktur, eines Peptids, Peptidfragments und/oder Peptidanalogons, insbesondere einer Peptiden und/oder Peptidfragmenten entsprechenden Struktur, als Sonde an das mindestens eine Aggregat bestimmt wird, bevor eine Selbstaggregation der mindestens einen Sonde überwiegt.

Es stellt generell eine Methode dar, die es insbesondere ermöglicht, Substanzen, insbesondere umfassend Proteine, Proteinfragmente, Proteinanaloga, insbesondere Proteinen und/oder Proteinfragmenten entsprechende Strukturen, Peptide, Peptidfragmente und/oder Peptidanaloga, insbesondere Peptiden und/oder Peptidfragmenten entsprechende Strukturen, insbesondere synthetisch hergestellte Peptide und gentechnisch synthetisierte Proteine, auf Kontaminationen mit Aggregaten zu untersuchen.

Die Aggregate gehören bevorzugt demselben Typ von Proteinen, Proteinfragmenten, Proteinanaloga, insbesondere Proteinen und/oder Proteinfragmenten entsprechenden Strukturen, Peptiden, Peptidfragmenten und/oder Peptidanaloga, insbesondere Peptiden und/oder Peptidfragmenten entsprechenden Strukturen, wie die Substanz, an.

Das erfindungsgemäße Verfahren eignet sich sowohl zu einer groben ersten, qualitativen Abschätzung als auch zu einer quantitativen Konzentrationsbestimmung der Aggregate. Es erlaubt somit eine hoch sensitive Qualitätskontrolle auf Aggregatverunreinigungen, wie sie bisher nicht möglich ist.

Daher ist das erfindungsgemäße Verfahren insbesondere auch zur Qualitätskontrolle von Therapeutika, Prozeßvalidierung von biotechnologisch und/oder chemisch synthetisierten und/oder isolierten nativen oder synthetischen Proteinen, Proteinfragmenten, Proteinanaloga, insbesondere Proteinen und/oder Proteinfragmenten entsprechenden Strukturen, Peptiden, Peptidfragmenten und/oder Peptidanaloga, insbesondere Peptiden und/oder Peptidfragmenten entsprechenden Strukturen geeignet.

Es ist mit Hilfe des erfindungsgemäßen Verfahrens sogar der Nachweis von "inclusion bodies", die bei der Exprimierung von Proteinen in Prokaryoten entstehen, möglich.

Ferner ermöglicht es die Überwachung von Langzeiteffekten bei der Lagerung von Substanzen die beispielsweise Proteine, Proteinfragmente, Proteinanaloga, insbesondere Proteinen und/oder Proteinfragmenten entsprechende Strukturen, Peptide, Peptidfragmente und/oder Peptidanaloga, insbesondere Peptiden und/oder Peptidfragmenten entsprechende Strukturen enthalten und eine Optimierung der Produktstabilität.

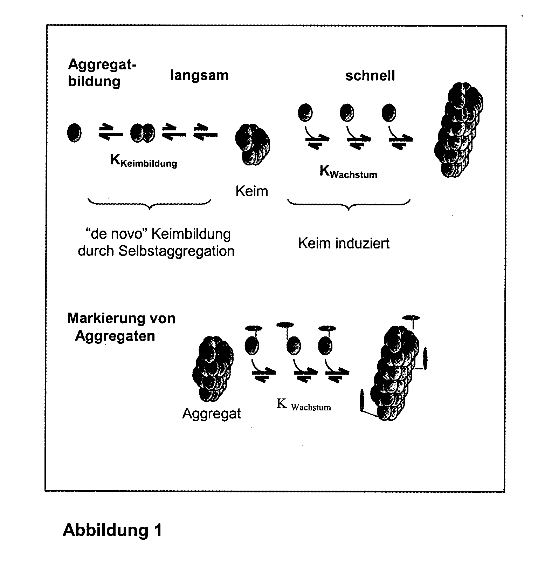

Das Prinzip des erfindungsgemäßen Verfahrens ist in Abbildung 1 dargestellt. Das erfindungsgemäße Verfahren nutzt den Ablauf des Prozesses der Aggregatbildung aus. Der Prozeß beginnt zunächst mit einer langsamen sog. "keimbildenden" Reaktion. Hierbei lagern sich einzelne Monomere, insbesondere Proteine, Proteinfragmente, Proteinanaloga, insbesondere Proteinen und/oder Proteinfragmenten entsprechende Strukturen, Peptide, Peptidfragmente und/oder Peptidanaloga, insbesondere Peptiden und/oder Peptidfragmenten entsprechende Strukturen, zu kleinen, wenige Monomere enthaltenden, Aggregaten, sog. Keimen, zusammen (Selbstaggregation/"de novo" Aggregation). Dabei liegt das Gleichgewicht deutlich auf der Seite der Monomere. Im zweiten Schritt dagegen, bei dem in einer schnellen Reaktion weitere Monomere an den Keim angelagert werden, liegt das Gleichgewicht auf Seiten des Produktes. Diesen Prozeß nennt man "Keim induzierte Multimerisierung". Es entstehen große Aggregate.

Die Schnelligkeit der zweiten Reaktion wird bei der Bestimmung von Aggregaten mittels des erfindungsgemäßen Verfahrens ausgenutzt.

Gemäß des erfindungsgemäßen Verfahrens erfolgt die Bestimmung von Aggregaten, die insbesondere aus Proteinen, Proteinfragmenten, Proteinanaloga, insbesondere Proteinen und/oder Proteinfragmenten entsprechenden Strukturen, Peptiden, Peptidfragmenten und/oder Peptidanaloga, insbesondere Peptiden und/oder Peptidfragmenten entsprechenden Strukturen bestehen, mit Hilfe von Sonden, die einen Aggregationsprozeß unterstützen können, insbesondere mindestens einer Sonde, die die Aggregate erkennt und an diese bindet.

Erfindungsgemäß kann es bevorzugt sein, dass die Aggregate in flüssiger Phase nachgewiesen werden. Es kann daher wünschenswert sein, dass sich eine auf Aggregate zu untersuchende Substanz, vor Bestimmung der Assoziation der mindestens einen Sonde an mindestens eine Aggregat, in einer Flüssigkeit befindet oder in eine Flüssigkeit eingebracht wird.

Vorzugsweise ist die mindestens eine Sonde eine monomere oder oligomere Einheit von Proteinen, Proteinfragmenten, Proteinanaloga, insbesondere Proteinen und/oder Proteinfragmenten entsprechenden Strukturen, insbesondere Antikörpern, Peptiden, Peptidfragmenten und/oder Peptidanaloga, insbesondere Peptiden und/oder Peptidfragmenten entsprechenden Strukturen.

Die Sonde kann erfindungsgemäß zu dem gleichen (homologe Detektion) oder einem anderen Typ (heterologe Detektion) der monomeren oder oligomeren Einheiten, insbesondere Proteinen, Proteinfragmenten, Proteinanaloga, insbesondere Proteinen und/oder Proteinfragmenten entsprechende Strukturen, Peptiden, Peptidfragmenten und/oder Peptidanaloga, insbesondere Peptiden und/oder Peptidfragmenten entsprechende Strukturen des mindestens einen Aggregats gehören. So kann z. B. die Sonde ein bestimmter Antikörper sein, wobei die Aggregate aus demselben (homologe Detektion) oder einem anderen Antikörper (heterologe Detektion) bestehen.

Es ist daher ebenfalls bevorzugt, wenn die Sonde zu dem gleichen (homologe Detektion) oder einem anderen Typ (heterologe Detektion) der monomeren oder oligomeren Einheiten, insbesondere Proteine, Proteinfragmente, Proteinanaloga, insbesondere Proteinen und/oder Proteinfragmenten entsprechende Strukturen, Peptide, Peptidfragmente und/oder Peptidanaloga, insbesondere Peptiden und/oder Peptidfragmenten entsprechende Strukturen, der mindestens einen zu untersuchenden Substanz, gehört.

Insbesondere durch den Einsatz von zu den Aggregaten homologen Proteinen, Proteinfragmenten, Proteinen und/oder Proteinfragmenten entsprechenden Strukturen, insbesondere Antikörpern, Peptiden, Peptidfragmenten und/oder Peptiden und/oder Peptidfragmenten entsprechenden Strukturen als Sonden oder den kombinierten Einsatz mit anderen Sonden, wie spezifische Antikörper, können falsch positive Detektionen von Aggregaten und somit beispielsweise eine ungenaue Quantifizierung insbesondere in biologischen Medien unterdrückt werden.

Allerdings ist es erfindungsgemäß insbesondere bei der qualitativen Bestimmung von Aggregaten wünschenswert, dass die mindestens eine Sonde heterolog zu monomeren oder oligomeren Einheiten des mindestens einen Aggregats und/oder der Substanz ist. Ein Beispiel dafür ist die Bestimmung von Aggregaten mit Antikörpern unter Ausnutzung der Antigen-Antikörper-Wechselwirkungen.

Ein Vergleich von heterologer mit homologer Detektion ist an dem Beispiel von Antikörpern in Abbildung 5 dargestellt. Hier zeigt der Nachweis der Aggregate über homologe bzw. heterologe Bindung vergleichbare Ergebnisse. Generell weisen die untersuchten Antikörperchargen allerdings sehr unterschiedliche Aggregatgehalte auf.

Damit gewährleistet ist, dass die Sonden als Monomere vorliegen oder auch zur Herstellung einer aggregatfreien Lösung, kann erfindungsgemäß eine Behandlung der mindestens einen Sonde und/oder der Aggregate, insbesondere der Proteine, Proteinfragmente, Proteinanaloga, insbesondere Proteinen und/oder Proteinfragmenten entsprechenden Strukturen, insbesondere Antikörpern, Peptide, Peptidfragmente und/oder Peptidanaloga, insbesondere Peptiden und/oder Peptidfragmenten entsprechenden Strukturen mit physikalischen Methoden wie Ultraschall, Einwirkung von Temperaturänderungen, Zentrifugation, chemischen Methoden, wie Behandlung mit Lösungen unterschiedlicher Ionenstärke, Behandlung mit Lösungen chaotroper Ionen, Behandlung mit Detergenzien und/oder Enzymen, insbesondere Proteasen durchgeführt werden.

Erfindungsgemäß ist es wünschenswert, wenn die mindestens eine Sonde und/oder das mindestens eine Aggregat, mindestens eine detektierbare Eigenschaft aufweisen. Es ist dabei bevorzugt, dass die mindestens eine detektierbare Eigenschaft entweder bereits in der mindestens einen Sonde und/oder dem mindestens einen Aggregat, insbesondere in den Proteinen, Proteinfragmenten, Proteinanaloga, insbesondere Proteinen und/oder Proteinfragmenten entsprechenden Strukturen, insbesondere Antikörpern, Peptiden, Peptidfragmenten und/oder Peptidanaloga, insbesondere Peptiden und/oder Peptidfragmenten entsprechenden Strukturen, enthalten ist oder nachträglich eingeführt wird.

Als detektierbare Eigenschaften kommen beispielsweise deren Größe und Ausdehnung in Betracht. Aber auch Eigenschaften wie Molmasse, Struktur messbar durch Zirkular-Dichroismus, optische Eigenschaften wie Lumineszenz, insbesondere Fluoreszenz oder Absorption, aber auch Radioaktivität können zur Bestimmung der Assoziation der mindestens einen Sonde und/oder des mindestens einen Aggregats herangezogen werden.

Die detektierbaren Eigenschaften werden erfindungsgemäß beispielsweise auch durch Fluoreszenzmarkierungen, die niedermolekulare, aber auch hochmolekulare Gruppen sein können, hergestellt. So können beispielsweise markierte Antikörper, die an die Aggregate gebunden werden, diese markieren. Die Assoziation der Sonden kann dann entsprechend vermessen werden. Es können verschiedenfarbige Markierungen unterschiedlicher Sonden vorgenommen werden und an diesen eine Koinzidenzanalyse beispielsweise durch Kreuzkorrelation vorgenommen werden. Es ist ebenfalls möglich, die Sonden mit entsprechenden Markierungen zu versehen, die entweder niedermolekulare fluoreszierende Liganden und/oder entsprechende Markierungskonjugate sind.

Es ist erfindungsgemäß bevorzugt, dass die mindestens eine detektierbare Eigenschaft spezifisch für die mindestens eine Sonde und/oder das mindestens eine Aggregat ist.

Einer zu untersuchenden Substanz werden erfindungsgemäß daher mindestens eine monomere oder oligomere Sonde, in einer bevorzugten Form des erfindungsgemäßen Verfahrens eine markierte monomere Sonde, die eine detektierbare Eigenschaft aufweist, zugesetzt. Enthält die zu untersuchende Substanz Aggregate (Keime), so lagern sich die Sonden an diese an und es entstehen große, insbesondere markierte, Aggregate.

Dabei ist es wünschenswert, dass mindestens eine detektierbare Eigenschaft bei der Assoziation der mindestens einen Sonde und dem mindestens einen Aggregat eine Änderung erfährt.

Es ist ebenfalls möglich, dass bereits die Aggregate (Keime), insbesondere durch eine intrinsische detektierbare Eigenschaft eine Markierung aufweisen, in so einem Fall ist es wünschenswert Sonden ohne detektierbare Eigenschaften oder mit einer anderen detektierbaren Eigenschaft zu verwenden.

Erfindungsgemäß erfolgt die Bestimmung der mindestens einen detektierbaren Eigenschaft und/oder ihrer Änderung bevorzugt mittels physikalischer Methoden, insbesondere spektroskopischer Methoden.

Als mögliche Methoden zur Bestimmung der mindestens einen detektierbaren Eigenschaft der Sonden oder Aggregate werden bevorzugt fluorimetrische Verfahren, wie konfokale Fluoreszenzspektroskopie, Fluoreszenzkorrelationsspektroskopie (FCS), FCS in Kombination mit Kreuzkorrelation, FIDA (Fluorescence intensity distribution analysis), FIMDA, FILDA, cFLA sowie 2D-FIDA mit den jeweils zugehörigen Auswertungsverfahren, angewendet.

Die Kombination mit extrem sensitiven Nachweisverfahren mittels konfokaler Optik, beipielsweise fluorimetrische Methoden, wie FIDA (Fluorescence intensity distribution analysis), FIMDA oder beispielsweise FILDA führt zu einer entsprechenden Verstärkung des Messsignals. Beispielsweise werden bei Anwendung des FIDA-Nachweisverfahrens die Moleküle hinsichtlich ihrer spezifischen Helligkeiten unterschieden. Diese Methode ist zudem molekulargewichtsunabhängig (PCT/EP97/05619; Kask et al. 1999 Procl. Natl. Sci. USA. 96, 13756). Erfindungsgemäß werden so beispielsweise die Helligkeitsunterschiede zwischen den kleinen, wenig leuchtenden Monomeren und den großen intensiv fluoreszierenden Aggregaten bestimmt. Die Messung erfolgt hier unmittelbar nach Zugabe der Sonde zur Probe. Dadurch ist sichergestellt, daß die gemessenen Aggregate bereits als Keime in der Testlösung vorlagen und nicht durch Selbstaggregation der Sonde entstanden sind.

Als Detektionsparameter, die aus den detektierbaren Eigenschaften der Sonden oder Aggregate und/oder einer Änderung der detektierbaren Eigenschaften ermittelt werden, sind Translationsdiffusionsgeschwindigkeiten, Lebensdauer angeregter Zustände, Polarisation von Strahlung, Energietransfer, Quantenausbeute, molekulare Helligkeiten, Partikelzahl oder Konzentration sowie Intensitätsdifferenzen zu nennen.

Wird beispielsweise die Translationsdiffusionsgeschwindigkeit ermittelt, so ist es bei langsam diffundierenden Komplexen aus Sonden und Aggregat wünschenswert, diese mittels eines Scanningprozesses zu erfassen.

Tritt allerdings die Assoziation der Sonde an Aggregate (Keime) gegenüber der Selbstaggregation der Sonde in den Hintergrund, ist eine sichere Messung nicht mehr gewährleistet. Es sind daher relativ kurze Messzeiten im Sekunden- bis Stundenbereich nötig. Die Zeiten, in denen die Messungen durchgeführt werden, hängen naturgemäß von den jeweiligen Messbedingungen ab, sind jedoch in Vorversuchen einfach zu ermitteln. Als Parameter, die die Messzeit beeinflussen können, sind insbesondere Konzentration des Aggregats (Keims) oder der Sonde zu nennen. Sind beispielsweise die Sondenkonzentrationen vergleichsweise hoch gegenüber der Aggregatkonzentration, wird die Selbstaggregation der Sonden eher einsetzen, als dies der Fall wäre, wenn die Sonden in geringer Konzentration vorhanden sind oder gar die Sondenkonzentration in ähnlicher Größenordnung wie die Aggregatkonzentration oder darunter liegt.

Generell sind mit dem erfindungsgemäßen Verfahren Meßzeiten von weit unter einer Stunde, insbesondere weniger als 30 Minuten, möglich. In der Regel werden die Messungen sogar innerhalb von wenigen Sekunden, insbesondere 1 bis 60 Sekunden, bevorzugt 20 bis 40 Sekunden, besonders bevorzugt 30 Sekunden durchgeführt. Dies ist besonders vorteilhaft für die Verwendung der Methode im Routinebetrieb, insbesondere in der Qualitätskontrolle.

Zur Erhöhung der Sensitivität des Verfahrens und Steigerung der Analysengeschwindigkeit kann es generell gewünscht sein, die Probe mittels eines durch die Meßlösung bewegten konfokalen Volumenelementes "abzurastern".

Zudem können mit dem erfindungsgemäßen Verfahren noch Konzentrationen an Aggregaten (Keimen) bis zu s 0.3fM qualitativ nachgewiesen und quantitativ bestimmt werden. Das erfindungsgemäße Verfahren weist somit gegenüber den bekannten Methoden eine um bis zu einem Faktor 1000 erhöhte Sensitivität auf.

Darüber hinaus werden nur geringe Meßvolumina von weniger als < 100 µl benötigt, wobei bevorzugt Volumina von < 20 µl eingesetzt oder sogar < 10 µl verwendet werden.

Die Aggregate können zudem direkt als intakte Aggregate detektiert werden und müssen nicht erst aufgebrochen und in ihre einzelnen Komponenten gespalten werden. Aufwendige Präparationen und Probenvorbereitungsmethoden sind nicht notwendig bzw. werden vermieden, um die zu analysierende Lösung möglichst im Ursprungszustand (nativ) untersuchen zu können (Artefaktvermeidung).

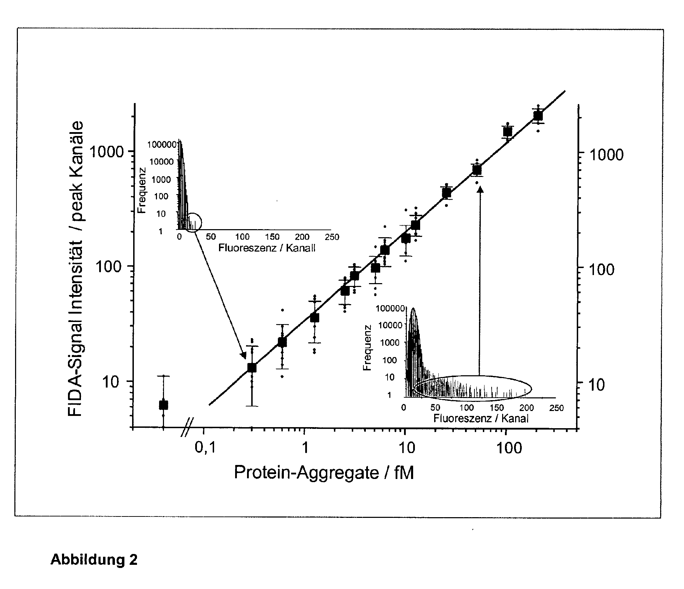

Abbildung 2 zeigt als Beispiel für das erfindungsgemäße Verfahren, die Anwendung der FIDA-Detektion in Abhängigkeit von der Aggregatkonzentration. Es ist deutlich zu erkennen, dass selbst in Konzentrationsbereichen von weniger als 1 fM, sogar noch weniger als 0.3 fM, deutliche und spezifische Signale zu erhalten sind.

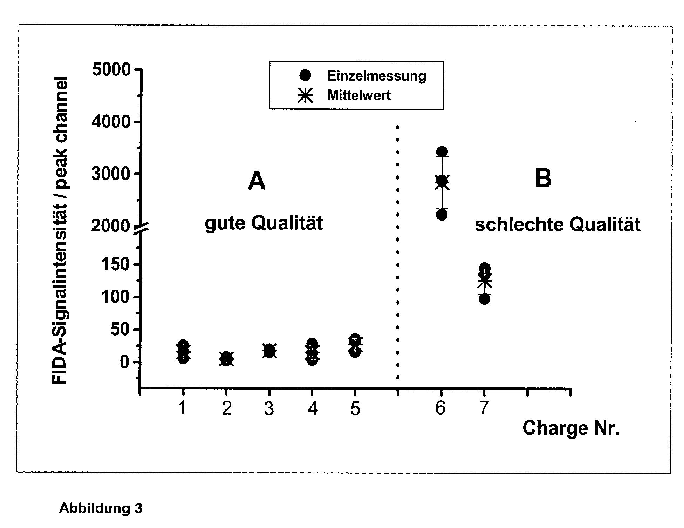

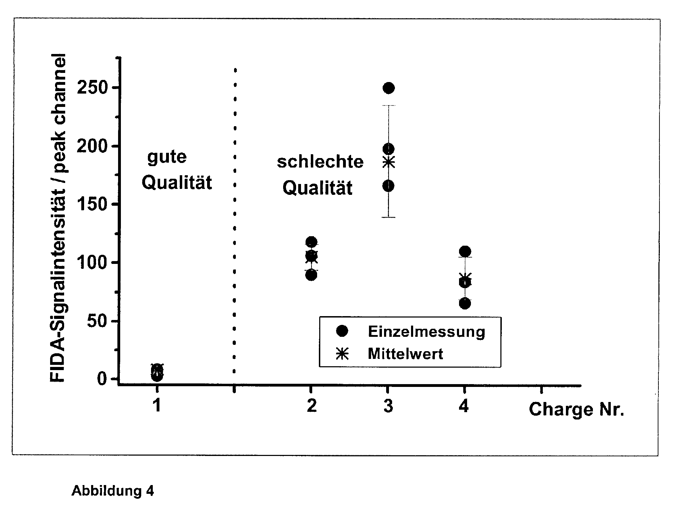

Abbildung 3 und 4 zeigen die Anwendung des erfindungsgemäßen Verfahrens zur Untersuchung der Qualität von verschiedenen Peptid- bzw. Antikörperchargen. Es ist deutlich zu erkennen, dass verschiedene Chargen von demselben Hersteller oder auch unterschiedlicher Hersteller große Unterschiede im Aggregatgehalt und somit in der tatsächlichen Konzentration an aktivem Peptid- oder Antikörper-Monomer aufweisen.

Das erfindungsgemäße Verfahren läßt sich auch zur Bestimmung von Substanzen, die eine Aggregatbildung verhindern oder bereits gebildete Aggregate sogar wieder auflösen können, einsetzen. Solche Substanzen, sogenannte Stabilisatoren, können insbesondere die Lagerfähigkeit insbesondere von Proteinen, Proteinfragmenten, Proteinanaloga, insbesondere Proteinen und/oder Proteinfragmenten entsprechenden Strukturen, insbesondere Antikörpern, Peptiden, Peptidfragmenten und/oder Peptidanaloga, insbesondere Peptiden und/oder Peptidfragmenten entsprechenden Strukturen erhöhen und sind auch für die Stabilisierung von insbesondere Therapeutika wichtig. Generell eignen sich Substanzen, wie beispielsweise Detergenzien, Lösungsmittel, Proteine, Peptide, Serumalbumin. Allerdings besteht gerade im Hinblick auf eine Anwendung in Medikamenten/Therapeutika das Problem, dass diese häufig nicht im menschlichen oder tierischen Organismus einsetzbar sind, wie z. B. DMSO oder SDS oder sie beinhalten, wie beispielsweise Serumalbumin die Möglichkeit der Übertragung von Krankheiten.

Es kann auch wünschenswert sein Substanzen zu finden, die den Prozess der Aggregatbildung beschleunigen und den Aggregatgehalt erhöhen, beispielsweise zur Aufreinigung, Aufkonzentrierung und/oder Abtrennung von zur Aggregation neigenden Molekülen, insbesondere Proteinen, Proteinfragmenten, Proteinanaloga, insbesondere Proteinen und/oder Proteinfragmenten entsprechenden Strukturen, insbesondere Antikörpern, Peptiden, Peptidfragmenten und/oder Peptidanaloga, insbesondere Peptiden und/oder Peptidfragmenten entsprechenden Strukturen.

Sollen beispielsweise Moleküle, die nur in geringer Konzentration in einer Lösung vorhanden sind, aufkonzentriert werden, kann erfindungsgemäß mindestens ein solcher Induktor, beispielsweise ein Metallionen, zugesetzt werden, der eine Aggregation dieser Moleküle bewirkt. Die Aggregate lassen sich nun durch herkömmliche physikalische und/oder chemische Trennmethoden, beispielsweise Zentrifugation und/oder HPLC, aus der Lösung abtrennen und können dann erneut in einem geringeren Volumen, gelöst werden. Zur vollständigen Lösung dieser Aggregate kann es wünschenswert sein, physikalische Methoden wie Ultraschall, Einwirkung von Temperaturänderungen und/oder chemische Methoden einzusetzen.

Die Identifizierung und Optimierung von Substanzen, die eine Aggregatbildung beeinflussen können, insbesondere Induktoren und/oder Stabilisatoren, kann erfindungsgemäß folgendermaßen durchgeführt werden:

- Bereitstellen mindestens einer aggregatfreien Lösung und/oder einer Lösung mit bekanntem Aggregatgehalt, insbesondere enthaltend Proteine, Proteinfragmente, Proteinanaloga, insbesondere Proteinen und/oder Proteinfragmenten entsprechende Strukturen, Peptide, Peptidfragmente und/oder Peptidanaloga, insbesondere Peptiden und/oder Peptidfragmenten entsprechende Strukturen.

- Einbringen mindestens einer, potentiell eine Aggregatbildung beeinflussenden, Substanz.
- Mischung der mindestens einen Lösung.
- Bestimmung des Aggregatgehaltes mit Hilfe des erfindungsgemäßen Verfahrens zur qualitativen und/oder quantitativen Bestimmung von Aggregaten.
- Bestimmung der Aggregatbildung beeinflussenden Eigenschaften der mindestens einen Substanz durch Vergleich des Aggregatgehaltes dieser Lösung mit dem mindestens einer Referenzprobe.

Es kann erfindungsgemäß bevorzugt sein, nach der Mischung der Lösung eine Inkubation der Lösung durchzuführen. Die Inkubationsbedingungen, insbesondere Inkubationszeit und -temperatur, können jeweils den Versuchsbedingungen angepasst werden.

Zur Herstellung einer aggregatfreien Lösung, kann es erfindungsgemäß wünschenswert sein, die Lösung physikalischen Methoden wie Ultraschall, Einwirkung von Temperaturänderungen, Zentrifugation und/oder chemischen Methoden auszusetzen oder der Lösung Aggregate auflösende Substanzen, insbesondere Detergenzien, Enzyme, insbesondere Proteasen und/oder Lösungen unterschiedlicher Ionenstärke und/oder chaotroper Ionen, beizufügen.

Bei der Referenzprobe handelt es sich bevorzugt um eine aggregatfreie Lösung und/oder Lösung mit bekanntem Aggregatgehalt desselben Proteins, Proteinfragments, Proteinanalogons, insbesondere Proteins und/oder Proteinfragments entsprechende Struktur, Peptids, Peptidfragments und/oder Peptidanalogons, insbesondere Peptid und/oder Peptidfragment entsprechende Struktur, vor Zugabe der mindestens einen Aggregatbildung beeinflussenden Substanz.

Es ist ebenfalls wünschenswert, dass die Referenzprobe inkubiert wird. Vorzugsweise sind die Inkubationsbedingungen (Zeit, Temperatur) der Referenzprobe mit denen der Lösung identisch.

Erfindungsgemäß ist bei Zugabe einer eine Aggregatbildung verringernde Substanz, sogenannte Stabilisatoren, ein signifikant niedrigerer Aggregatgehalt der Lösung im Vergleich zur Referenzprobe festzustellen.

Erfindungsgemäß umfasst der Begriff "Aggregatbildung verringernde Substanz" auch Substanzen, die eine Aggregatbildung vollständig hemmen.

Substanzen, die Aggregate auflösen können, bewirken nach Zugabe eine im Vergleich zur Referenzprobe signifikante Erniedrigung des Aggregatgehalts der Lösung.

Diese Aggregate auflösende Substanzen zeichnen sich auch dadurch aus, dass sie nach Zugabe eine im Vergleich zu einer Referenzprobe, die eine Aggregatbildung verringernde Substanz enthält, signifikante Erniedrigung des Aggregatgehaltes der Lösung bewirken.

Die Zugabe einer Aggregatbildung beschleunigenden Substanz, sogenannte Induktoren, bewirkt eine signifikante Erhöhung des Aggregatgehaltes der Lösung im Vergleich zur Referenzprobe.

Generell kann es wünschenswert sein, in einer Versuchsreihe verschiedene Konzentrationen der potentiellen die Aggregatbildung beeinflussenden Substanz zu untersuchen.

Erfindungsgemäß kann es sich bei der mindestens einen, die Aggregatbildung beeinflussen Substanz insbesondere um ein Detergenz, eine chaotrope Substanz, Salz, Lipid, Lipidderivat, Nukleinsäure, Nukleinsäurederivat, Kohlenhydrat bzw. dessen Derivat, Lösungsmittel, Protein, Proteinfragment, Proteinanalogon, insbesondere Protein und/oder Proteinfragment entsprechende Struktur, Peptid, Peptidfragment und/oder Peptidanalogon, insbesondere Peptid und/oder Peptidfragment entsprechende Struktur handeln.

Das erfindungsgemäße Verfahren wird durch die nachfolgenden Beispiele näher erläutert.

**Beispiel 1:**

**Qualitätskontrolle verschiedener Aβ-Peptidchargen**

*Verschiedene Peptidchargen werden auf das Vorhandensein von Aggregaten getestet.*

Material:

- Verschiedene Aß-Peptidchargen unterschiedlicher Hersteller:

- Aβ1-42 Fa. Bachem, Lot Nr. 516817
- Aβ1-42 Fa. Calbiochem, Lot Nr. D04765-4
- Aβ1-42 Lieferung vom 13.09.1999 mit Fmoc Schutzgruppe, #2301, synthetisiert von Margit Ellis, AG Biopolymere, Centre for Molecular Biology, University of Heidelberg,
  Aliquotweise Abspaltung der Schutzgruppe, Untersuchung dreier verschiedener Aliquots
- Aβ1-42 Lieferung vom 18.08.1999 mit Fmoc-Hec1 Schutzgruppe, synthetisiert von Dr. Peter Henklein, Medizinische Fakultät (Charité), Institut für Biochemie d. Humboldt Universität Berlin
- Aβ18-42 + 0,5 nmol Nicotin, SD-06.08 vom 6.7.1999 synthetisiert von Evotec OAI, Labeling Chemistry
- DMSO, Fa. Sigma, Best. Nr. D-8779, Lot Nr. 38H3447
- Na-Phosphatpuffer (0,02M; pH 7,0) => (Lsg. 1)
- Aß1-42 Peptid (Lieferung 13.09.1999 mit Fmoc Schutzgruppe, #2301, synthetisiert von Margit Ellis, AG Biopolymere, Centre for Molecular Biology, University of Heidelberg) fluoreszenzmarkiert mit EVOblue[30] (KS-01-06, vom 09.05.2000, Evotec OAI) in 0,02M Na-Phosphatpuffer pH 7,0 mit 0,2% SDS => (Lsg. 2) Endkonzentration Peptid 50 nM

Durchführung:

Die hier getesteten Peptide sind stark hydrophob und daher schlecht in Puffer löslich. Aus diesem Grunde werden sie zunächst in DMSO gelöst. Die zu testenden Peptide werden jeweils 1mM in DMSO gelöst. Hiervon ausgehend ergibt sich folgende Verdünnungsreihe:

→ 1:1 in 50% DMSO (=> 500μM)
→ 1:50 in Lsg. 1 (=> 10μM)

Meßansatz:

19μl Peptidlösung (10μM)

+ 1μl Lsg 2

Der Meßansatz wird gut durchmischt, in eine Kammer des Probenträgers appliziert und mittels FIDA vermessen. Es werden jeweils 10 Messungen à 30 sec durchgeführt.
Als Kontrolle wird 1μl Lsg 2 in 19μl Lsg1 vermessen.

Ergebnis:

In Abbildung 3 sind die Ergebnisse der Versuchsreihe graphisch dargestellt. Es ist eine deutliche Unterscheidung zwischen (a) aggregatfreien Chargen (gute Qualität) und (b) aggregathaltigen Chargen (schlechte Qualität) erkennbar. Während in guten Chargen nur das niedrige Fluoreszenzsignal der Monomere zu sehen ist, sind die schlechten Chargen durch das Vorhandensein von großen fluoreszenten Aggregaten charakterisiert. Letzteres zeigt sich in der deutlich erhöhten FIDA-Signalintensität.

**Beispiel 2:**

**Qualitätskontrolle verschiedener Antikörperproduktionschargen**

*Antikörper verschiedenster Typen und Chargen werden auf das Vorhandensein von Aggregaten getestet.*

Materialien:

- Na-Phosphatpuffer (0,02M; pH 7,0) => (Lsg. 1)

- Antikörperchargen der folgenden Hersteller:

- Anti-Aß 6E10 monoclonal antibody, IgG1, Produktnummer: 320-02, Lot Nr.: 306, Fa. Signet Pathology
- Anti-alpha-Synuclein (human, monodonal) 15G7, Rat IgG2a, Produkt Nr.: 804-258-L001, Batch Nr. L05992, Fa. Alexis Biochemicals
- Anti-alpha-Synuclein (rabbit, polyclonal), Katalog Nr.: 44-444, Lot Nr.: 0101, Fa. Biosource International
- Anti-CystatinC Antikörper (rabbit anti-human), Code Nr.: A 0451, Lot Nr.: 028, Fa. DAKO

- Verdünnung der einzelnen Antikörper in Lösung 1, sodaß sich eine Antikörperkonzentration von 500 μg/ml ergibt.

- mit EVOblue[30] (KS-01-06, vom 09.05.2000, Evotec OAI) fluoreszenzmarkierter Antikörper der jeweils zu testenden Charge in 0,02M Na-Phosphatpuffer pH 7,0 mit 0,2% SDS => (Lsg. 3) Endkonzentration Antikörper 50 nM (Ein Teil der jeweils zu testenden Antikörpercharge wird fluoreszenzmarkiert und dient dann als Detektions-Sonde.)

Durchführung:

Meßansatz:

18 μl Lsg. 1

+ 1μl Antikörper

+ 1μl Lsg. 3

Der Meßansatz wird gut durchmischt, in eine Kammer des Probenträgers appliziert und mittels FIDA vermessen. Es werden jeweils 10 Messungen à 30 sec durchgeführt.
Als Kontrolle wird 1μl Lsg 3 in 19μl Lsg1 vermessen.

Ergebnis:

Abbildung 4 zeigt die Ergebnisse der Versuchsreihe. Es sind zwei verschiedene Gruppen von Antikörperchargen zu erkennen. Die eine Charge mit guter Qualität (keine Aggregate) ist durch niedrige FIDA-Signalintensitäten gekennzeichnet. In den Chargen schlechter Qualität führen Aggregatverunreinigungen zu einem deutlichen Anstieg der FIDA-Signalintensität.

**Beispiel 3:**

**Heterologe und homologe Detektion von Antikörperaggregaten**

*Mit Hilfe einer fluoreszenzmarkierten Antikör-*

*personde lassen sich sowohl Aggregate derselben Charge (homologe Bindung) als auch Aggregate verschiedener Antikörperchargen (heterologe Bindung) nachweisen. Drei verschiedene Antikörperchargen werden mit zwei verschiedenen fluoreszenzmarkierten Antiköpersonden vermessen.*

**Materialien:**

- Na-Phosphatpuffer (0,02M; pH 7,0) => (Lsg. 1)

- Antikörperchargen der Hersteller:

  - Anti-alpha-Synuclein, rabbit PAb, polyclonal, Kat. Nr. 44-444, Lot Nr. 0101, Fa. Biosource International, 0,1mg/0,2ml

  - Anti-β-amyloid42, rabbit PAb, Kat. Nr. 44-344, Lot Nr. 44344132, Fa. Biosource International (0,391 mg/ml)

  - Anti-rabbit IgG (whole molecule), developed in goat, Kat. Nr. R-2004, Lot Nr. 79H9240, Fa. Sigma

- mit EVOblue30 (KS-01-06, vom 09.05.2000, Evotec OAI) fluoreszenzmarkierter Antikörper (anti-alpha-Synuclein, rabbit PAb, polyclonal, Kat.Nr. 44-444, Lot Nr. 0101, Fa. Biosource International, 0,1mg/0,2ml) 1:5 in 0,02M Na-Phosphatpuffer pH 7,0 mit 0,2% SDS verdünnt => (Lsg. 4).

- mit EVOblue[30] (KS-01-06, vom 09.05.2000, Evotec OAI) fluoreszenzmarkierter Antikörper (Anti-rabbit IgG (whole molecule), developed in goat, Kat. Nr. R-2004, Lot Nr. 79H9240, Fa. Sigma) 1:100 in 0,02M Na-Phosphatpuffer pH 7,0 mit 0,2% SDS verdünnt => (Lsg. 5).

Durchführung:

Meßansatz:

13 µl Lsg. 1

+ 2 µl 8% SDS

+ 4 µl Antikörper

+ 1 µl Lsg. 4 bzw. Lsg. 5

Der Meßansatz wird gut durchmischt, in eine Kammer des Probenträgers appliziert und mittels FIDA vermessen. Es werden jeweils 10 Messungen à 30 sec durchgeführt.

Als Kontrolle wird gemessen:

17µl Lsg. 1

+ 2µl 8% SDS

+ 1µl Lsg. 4 bzw. Lsg. 5

Ergebnis:

Abbildung 5 zeigt die Ergebnisse der Messungen. Die drei untersuchten Antikörperchargen sind durch sehr unterschiedliche Aggregatgehalte charakterisiert. Der Nachweis der Aggregate über homologe bzw. heterologe Bindung zeigt vergleichbare Ergebnisse.
Homologe Bindung bedeutet, daß ein Teil einer Antikörpercharge fluoreszenzmarkiert wird und als Sonde zur Detektion der Aggregate in der restlichen Charge dient. Die Chargen A und C (graue Balken) wurden auf diese Weise untersucht. Charge A weist viele Aggregate auf, während Charge C fast aggregatfrei ist.
Bei der heterologen Bindung wird ein Antikörper fluoreszenzmarkiert und dient als Sonde für verschiedenste Antikörper Chargen. So wurden die Chargen A und B mit fluoreszenzmarkiertem Antikörper C detektiert und die Chargen B und C mit fluoreszenzmarkiertem Antikörper A.
Die zahlreichen Aggregate in Charge A sind auch über heterologe Bindung nachzuweisen. Charge C weist auch bei heterologer Detektion so gut wie keine Aggregate auf. Die Charge B, die mit zwei verschiedenen heterolog bindenden Sonden untersucht wurde, zeigt in beiden Fällen einen geringen Aggregatgehalt.

**Patentansprüche**

1. Verfahren zur qualitativen und/oder quantitativen Bestimmung von Aggregaten, **dadurch gekennzeichnet, dass** die Assoziation mindestens eines Proteins, Proteinfragments, Proteinanalogons, insbesondere einer Proteinen und/oder Proteinfragmenten entsprechenden Struktur, Peptids, Peptidfragments und/oder Peptidanalogons, insbesondere einer Peptiden und/oder Peptidfragmenten entsprechenden Struktur, als Sonde an mindestens ein Aggregat bestimmt wird, bevor eine Selbstaggregation der mindestens einen Sonde überwiegt.

2. Verfahren nach Anspruch 1, **dadurch gekennzeichnet, dass** das mindestens eine Aggregat aus Proteinen, Proteinfragmenten, Proteinanaloga, insbesondere Proteinen und/oder Proteinfragmenten entsprechenden Strukturen, Peptiden, Peptidfragmenten und/oder Peptidanaloga, insbesondere Peptiden und/oder Peptidfragmenten entsprechenden Strukturen, besteht.

3. Verfahren nach mindestens einem der Ansprüche 1 und/oder 2, **dadurch gekennzeichnet**, das die Aggregate in flüssiger Phase nachgewiesen werden.

4. Verfahren nach mindestens einem der Ansprüche 1 bis 3, **dadurch gekennzeichnet, dass** die mindestens eine Sonde homolog zu monomeren oder oligomeren Einheiten des mindestens einen Aggregats ist.

5. Verfahren nach mindestens einem der Ansprüche 1 bis 4, **dadurch gekennzeichnet, dass** die mindestens eine Sonde heterolog zu monomeren oder oligomeren Einheiten des mindestens einen Aggregats ist.

6. Verfahren nach mindestens einem der Ansprüche 1 bis 5, **dadurch gekennzeichnet, dass** die mindestens eine Sonde und/oder das mindestens eine Aggregat, mindestens eine detektierbare Eigenschaft aufweisen.

7. Verfahren nach mindestens einem der Ansprüche 1 bis 6, **dadurch gekennzeichnet, dass** die mindestens eine detektierbare Eigenschaft entweder bereits in der mindestens einen Sonde und/oder dem mindestens einen Aggregat enthalten ist oder nachträglich eingeführt wird.

8. Verfahren nach mindestens einem der Ansprüche 1 bis 7, **dadurch gekennzeichnet, dass** die mindestens eine detektierbare Eigenschaft spezifisch für die mindestens eine Sonde und/oder das mindestens eine Aggregat ist.

9. Verfahren nach Anspruch 1 bis 8 **dadurch gekennzeichnet, dass** die mindestens eine detektierbare Eigenschaft bei der Assoziation der mindestens einen Sonde und dem mindestens einen Aggregat eine Änderung erfährt.

10. Verfahren nach mindestens einem der Ansprüche 1 bis 9, **dadurch gekennzeichnet, dass** die mindestens eine detektierbare Eigenschaft Größe, Ausdehnung, Molmasse, Struktur, Zirkular-Dichroismus, optische Eigenschaften wie Lumineszenz, insbesondere Fluoreszenz, Absorption, Radioaktivität ist.

11. Verfahren nach mindestens einem der Ansprüche 1 bis 10, **dadurch gekennzeichnet, dass** die Bestimmung der mindestens einen detektierbaren Eigenschaft und/oder ihrer Änderung mittels physikalischer Methoden, insbesondere spektroskopischer Methoden, erfolgt.

12. Verfahren nach mindestens einem der Ansprüche 1 bis 11, **dadurch gekennzeichnet, dass** die Bestimmung der mindestens einen detektierbaren Eigenschaft und/oder ihrer Änderung mittels fluorimetrischer Methoden, wie konfokaler Fluoreszenzspektroskopie, Fluoreszenz-Korrelations-Spektrokopie (FCS), FCS in Kombination mit Kreuzkorrelation, Fluorescence Intensity Distribution Analysis (FIDA), FIMDA, FILDA, cFLA, 2D-FIDA, erfolgt.

13. Verfahren nach mindestens einem der Ansprüche 1 bis 12 **dadurch gekennzeichnet, dass** aus den detektierbaren Eigenschaften Parameter wie Translationsdiffusionsgeschwindigkeiten, Rotationsdiffusionsgeschwindigkeiten, Lebensdauern angeregter Zustände, Polarisation von Strahlung, Energietransfer, Quantenausbeute, molekulare Helligkeiten, Partikelzahl oder Konzentration, Intensitätsdifferenzen, ermittelt werden.

14. Verwendung des Verfahrens nach mindestens einem der Ansprüche 1 bis 13 zur Bestimmung des Aggregatgehaltes von Substanzen, enthaltend Proteine, Proteinfragmente, Proteinanaloga, insbesondere Proteinen und/oder Proteinfragmenten entsprechende Strukturen, Peptide, Peptidfragmente und/oder Peptidanaloga, insbesondere Peptiden und/oder Peptidfragmenten entsprechende Strukturen, insbesondere zur Qualitätskontrolle.

15. Verfahren zur Identifizierung und Optimierung von Substanzen, die eine Aggregatbildung beeinflussen können, mit folgenden Schritten:

   - Bereitstellen mindestens einer aggregatfreien Lösung und/oder einer Lösung mit bekanntem Aggregatgehalt, insbesondere enthaltend Proteine, Proteinfragmente, Proteinanaloga, insbesondere Proteinen und/oder Proteinfragmenten entsprechende Strukturen, Peptide, Peptidfragmente und/oder Peptidanaloga, insbesondere Peptiden und/oder Peptidfragmenten entsprechende Strukturen.
   - Einbringen mindestens einer, potentiell eine Aggregatbildung beeinflussenden, Substanz.
   - Mischung der mindestens einen Lösung.
   - Bestimmung des Aggregatgehaltes mit Hilfe des Verfahrens nach mindestens einem der Ansprüche 1 bis 13.
   - Bestimmung der Aggregatbildung beeinflussenden Eigenschaften der mindestens einen Substanz durch Vergleich des Aggregatgehaltes dieser Lösung mit dem mindestens einer Referenzprobe.

**16.** Verfahren gemäß Anspruch 15, **dadurch gekennzeichnet, dass** nach der Mischung eine Inkubation der Lösung durchgeführt wird.

**17.** Verfahren gemäß Anspruch 15 und/oder 16, **dadurch gekennzeichnet, dass** es sich bei der Referenzprobe, bevorzugt um eine aggregatfreie Lösung und/oder Lösung mit bekanntem Aggregatgehalt desselben Proteins, Proteinfragments, Proteinanalogons, insbesondere Proteins und/oder Proteinfragments entsprechende Struktur, Peptids, Peptidfragments und/oder Peptidanalogons, insbesondere Peptid und/oder Peptidfragment entsprechende Struktur, vor Zugabe der mindestens einen Aggregatbildung beeinflussenden Substanz, handelt.

**18.** Verfahren nach Anspruch 17, **dadurch gekennzeichnet, dass** die Referenzprobe ebenfalls inkubiert wird.

**19.** Verfahren nach mindestens einem der Ansprüche 15 bis 18, **dadurch gekennzeichnet, dass** eine Aggregatbildung verringernde Substanz einen signifikant niedrigeren Aggregatgehalt der Lösung im Vergleich zur Referenzprobe bewirkt.

**20.** Verfahren nach mindestens einem der Ansprüche 15 bis 19, **dadurch gekennzeichnet, dass** eine Aggregatbildung beschleunigende Substanz eine signifikante Erhöhung des Aggregatgehaltes im Vergleich zur Referenzprobe bewirkt.

**21.** Verfahren nach mindestens einem der Ansprüche 15 bis 20, **dadurch gekennzeichnet, dass** eine Aggregate auflösende Substanz eine signifikante Erniedrigung des Aggregatgehaltes der Lösung im Vergleich zur Referenzprobe und/oder einer Referenzprobe, die eine Aggregatbildung verringernde Substanz enthält, bewirkt.

**22.** Verfahren nach mindestens einem der Anprüche 15 bis 21, **dadurch gekennzeichnet, dass** es sich bei der mindestens einen Aggregatbildung beeinflussenden Substanz um ein Detergenz, eine chaotrope Substanz, Salz, Lipid, Lipidderivat, Nukleinsäure, Nukleinsäurederivat, Kohlenhydrat bzw. dessen Derivat, Lösungsmittel, Protein, Proteinfragment, Proteinanalogon, insbesondere Protein und/oder Proteinfragment entsprechende Struktur, Peptid, Peptidfragment und/oder Peptidanalogon, insbesondere Peptid und/oder Peptidfragment entsprechende Struktur handelt.

**Abbildung 1**

**Abbildung 2**

Abbildung 3

**Abbildung 4**

**Abbildung 5**

**Europäisches**
**Patentamt**

**EUROPÄISCHER RECHERCHENBERICHT**

Nummer der Anmeldung

EP 01 13 0633

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | WO 99 15903 A (EIGEN MANFRED ;BIESCHKE JAN (DE); PITSCHKE MARTIN (DE); POST KARIN) 1. April 1999 (1999-04-01) * das ganze Dokument * * Beispiele 1-10 * * Abbildungen 2,5 * --- | 1-13, 15-22 | G01N33/68 G01N33/58 G01N33/557 |
| A | FRANCO ROGNONI ET AL: "Influence of osmolarity and pH increase to achieve a reduction of monoclonal antibodies aggregates in a production process." CYTOTECHNOLOGY, Bd. 29, Nr. 1, 1999, Seiten 11-25, XP008005972 ISSN: 0920-9069 * Zusammenfassung * ----- | 14 | |

**RECHERCHIERTE SACHGEBIETE (Int.Cl.7)**

G01N

Der vorliegende Recherchenbericht wurde für alle Patentansprüche erstellt

| Recherchenort | Abschlußdatum der Recherche | Prüfer |
|---|---|---|
| DEN HAAG | 19. Juli 2002 | Tuynman, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**
EP 01 13 0633

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-07-2002

| Im Recherchenbericht angeführtes Patentdokument | Datum der Veröffentlichung | Mitglied(er) der Patentfamilie | Datum der Veröffentlichung |
|---|---|---|---|
| WO 9915903 A | 01-04-1999 | DE 19752712 A1 | 01-07-1999 |
| | | AU 9541398 A | 12-04-1999 |
| | | WO 9915903 A1 | 01-04-1999 |
| | | EP 1015888 A1 | 05-07-2000 |
| | | JP 2001517800 T | 09-10-2001 |
| | | US 2002042121 A1 | 11-04-2002 |

EPO FORM P0461

Für nähere Einzelheiten zu diesem Anhang : siehe Amtsblatt des Europäischen Patentamts, Nr.12/82